(19) Europäisches Patentamt
European Patent Office
Office européen des brevets

(11) EP 3 603 642 B1

(12) # EUROPEAN PATENT SPECIFICATION

(45) Date of publication and mention of the grant of the patent:
**10.01.2024 Bulletin 2024/02**

(21) Application number: **18775859.4**

(22) Date of filing: **27.03.2018**

(51) International Patent Classification (IPC):
***A61K 31/472*** (2006.01) ***A61K 9/08*** (2006.01)
***A61K 31/4725*** (2006.01) ***A61K 47/02*** (2006.01)
***A61P 27/02*** (2006.01) ***A61P 27/06*** (2006.01)
***A61K 9/00*** (2006.01) ***A61K 47/12*** (2006.01)
***A61K 47/18*** (2017.01) ***A61K 47/10*** (2017.01)
***A61K 47/34*** (2017.01) ***A61J 1/00*** (2023.01)
***A61K 31/5575*** (2006.01)

(52) Cooperative Patent Classification (CPC):
(C-Sets available)
**A61K 9/0048; A61J 1/00; A61K 9/08; A61K 31/472; A61K 31/4725; A61K 31/5575; A61K 47/02; A61K 47/10; A61K 47/12; A61K 47/186; A61K 47/34; A61P 27/02; A61P 27/06** (Cont.)

(86) International application number:
**PCT/JP2018/012403**

(87) International publication number:
**WO 2018/181294 (04.10.2018 Gazette 2018/40)**

(54) **PHARMACEUTICAL PREPARATION**
PHARMAZEUTISCHES PRÄPARAT
PRÉPARATION PHARMACEUTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**27.03.2017 JP 2017060582**
**27.03.2017 JP 2017060585**
**27.03.2017 JP 2017060586**
**28.04.2017 JP 2017089205**

(43) Date of publication of application:
**05.02.2020 Bulletin 2020/06**

(60) Divisional application:
**23204417.2 / 4 285 908**

(73) Proprietor: **Alcon Inc.**
**1701 Fribourg (CH)**

(72) Inventor: **YAMAKITA, Yoshihiro**
**Fuji-shi**
**Shizuoka 417-8650 (JP)**

(74) Representative: **Elkington and Fife LLP**
**Thavies Inn House**
**3-4 Holborn Circus**
**London EC1N 2HA (GB)**

(56) References cited:
**EP-A1- 2 127 638**
**EP-A1- 2 478 906**
**EP-A1- 3 590 513**
**EP-A1- 3 590 514**
**EP-A1- 3 590 515**
**WO-A1-2005/087237**
**WO-A1-2014/144781**
**WO-A1-2016/047720**
**JP-A- 2016 138 085**
**JP-A- 2016 515 520**
**US-A1- 2005 287 325**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 603 642 B1

- **ELLIS, EYDIE MILLER et al.: "Ocular hypotensive effect of the novel EP 3/FP agonist ONO-9054 versus Xalatan: results of a 28-day, double-masked, randomised study", British Journal of Ophthalmology, vol. 101, no. 6, 20 September 2016 (2016-09-20), pages 796-800, XP055538363, ISSN: 1468-2079**
- **LEVY, BRIAN et al.: "Ocular hypotensive safety and systemic absorption of AR -13324 ophthalmic solution in normal volunteers", American Journal of Ophthalmology, vol. 159, no. 5, May 2015 (2015-05), pages 980-985, XP055543404, ISSN: 0002-9394, DOI: doi:10.1016/j.ajo.2015.01.026**
- **REN, RUIYI et al.: "Netarsudil increases outflow facility in human eyes through multiple mechanisms", Investigative Ophthalmology and Visual Science, vol. 57, no. 14, November 2016 (2016-11), pages 6197-6209, XP055538365, ISSN: 1552-5783, DOI: doi:10.1167/iovs.16-20189**
- **Anonymous: "RHOPRESSA (netarsudil ophthalmic solution) Label, NDA 208254, Reference ID:4194833", NDA 208254, no. ID: 4194833, December 2017 (2017-12), pages 4-10, XP055612923,**


(52) Cooperative Patent Classification (CPC): (Cont.)

C-Sets
**A61K 31/472, A61K 2300/00;**
**A61K 31/4725, A61K 2300/00;**
**A61K 31/5575, A61K 2300/00**

**Description**

Technical Field

The present invention relates to the use of latanoprost for suppressing the content reduction of netarsudil or a salt thereof or a solvate thereof in a pharmaceutical preparation.

Background Art

Several isoquinoline-6-amino derivatives have hitherto been known to be useful in prevention and treatment of eye diseases such as ocular hypertension and glaucoma.

Specifically, for example, a compound represented by the following structural formula:

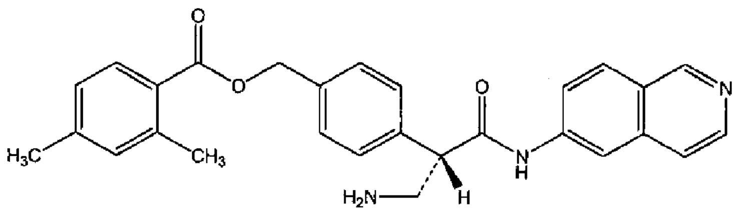

(Chemical Name: [4-[(2S)-3-amino-1-(isoquinolin-6-ylamino)-1-oxopropan-2-yl]phenyl]methyl 2,4-dimethylbenzoate), International Nonproprietary Name: netarsudil, hereinafter sometimes referred to as "netarsudil"), also known as AR-13324, has been reported to have pharmacological effects such as Rho kinase inhibitory effect and norepinephrine transporter inhibitory effect, and be useful in prevention and treatment of eye diseases such as ocular hypertension and glaucoma (for example, Patent Literature 1, and Non Patent Literatures 1 and 2).

Similarly, a compound represented by the following structural formula:

*rac*

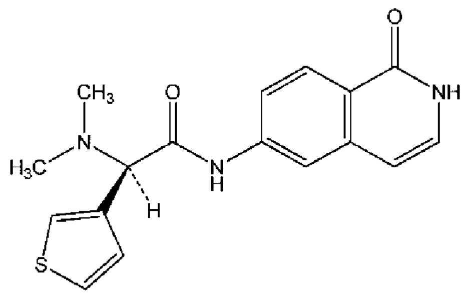

(Chemical Name: rac-(2R)-2-(dimethylamino)-N-(1-oxo-1,2-dihydroisoquinolin-6-yl)-2-(thiophen-3-yl) acetamide), International Nonproprietary Name: verosudil, hereinafter sometimes referred to as "verosudil"), also known as AR-12286, has been reported to have Rho kinase inhibitory effect and be useful in prevention and treatment of eye diseases such as ocular hypertension (for example, Patent Literature 2, and Non Patent Literatures 1 and 3).

Accordingly, it is very useful to establish a technique for stably formulating these isoquinolin-6-amino derivatives, for example, as an ophthalmic agent.

Meanwhile, Patent Literature 3 states that a composition containing netarsudil or verosudil was prepared in a 150 mL plastic container. However, in the literature, each composition was prepared for use in pharmacological studies. The literature does not describe the stability of netarsudil and verosudil in the composition at all, neither describes the material and characteristics of the container at all.

Citation List

Patent Literature

Patent Literature 1: JP-A-2012-525386
Patent Literature 2: WO2009/091898

Patent Literature 3: JP-A-2016-515520

Non Patent Literature

Non Patent Literature 1: Bacharach J. et al., Ophthalmology 122(2) 302-7 (2015)
Non Patent Literature 2: Sturdivant JM. et al., Bioorg Med Chem Lett. 26(10) 2475-80 (2016)
Non Patent Literature 3: Williams RD. et al., Am. J. Ophthalmol. 152(5) 834-41 (2011)

EP 3590513 A1, EP 3590514 A1, and EP 3590515 A1 disclose techniques for stably formulating an isoquinolin-6-amino derivative as an ophthalmic agent.

WO 2014/144781 A1 discloses compounds and compositions comprising an isoquinoline compound and a prostaglandin or a prostaglandin analog for treating glaucoma and/or reducing intraocular pressure.

SUMMARY OF THE INVENTION

An object of the present invention is to establish a technique for stably formulating an isoquinolin-6-amino derivative, namely netarsudil, as an ophthalmic agent.

Solution to Problem

An ophthalmic agent is usually a composition containing water (an aqueous composition). Thus, the present inventor prepared an aqueous composition comprising an isoquinolin-6-amino derivative such as netarsudil and housed the prepared composition in a container formed of a polyolefin resin to check its storage property. As a result, it was unexpectedly revealed that the content of the isoquinolin-6-amino derivative is reduced after storage at high temperature, which content reduction is caused presumably by adsorption to the resin.

Therefore, the present inventor conducted further extensive studies to suppress the content reduction of the isoquinolin-6-amino derivative in an aqueous composition. Consequently, the inventor has found that when the aqueous composition comprising the isoquinolin-6-amino derivative is adapted to further contain one or more components selected from the group consisting of an acid, a quaternary ammonium type surfactant, a polyhydric alcohol and a prostaglandin compound and housed in a container formed of a polyolefin resin, the content reduction is suppressed and a pharmaceutical preparation having improved stability can be obtained, and thus completed the present invention.

Namely, the present invention provides the use of latanoprost for suppressing the content reduction of netarsudil or a salt thereof or a solvate thereof in a pharmaceutical preparation comprising an aqueous composition comprising the following components (A) and (B) :

(A) netarsudil or a salt thereof or a solvate thereof; and
(B) latanoprost,

wherein the aqueous composition is housed in a container formed of polypropylene.

Effects of the Invention

According to the present invention, it is possible to improve the stability of isoquinolin-6-amino derivatives exemplified by netarsudil in an aqueous composition.

Description of Embodiments

<Component (A)>

In the present specification, component (A) is netarsudil or a salt thereof or a solvate thereof.

By the way, examples of the salt of the component (A) are not particularly limited as long as those are pharmaceutically acceptable salts. Specific examples of the salt include an inorganic acid salt such as a hydrochloride, a sulfate, a nitrate, a hydrofluoride, or a hydrobromide; an organic acid salt such as an acetate, a tartrate, a lactate, a citrate, a fumarate, a maleate, a succinate, a methanesulfonate, an ethanesulfonate, a benzenesulfonate, a toluenesulfonate, a naphthalenesulfonate, or a camphorsulfonate, and the hydrochloride and the methanesulfonate are preferred.

Examples of the solvate of the component (A) or a salt thereof include a hydrate and an alcohol solvate.

Furthermore, various stereoisomers can exist, but the configuration of component (A) is not particularly limited, and the compound may be a single stereoisomer or a mixture of various stereoisomers in any proportions.

In the specification, when an asymmetric carbon exists in a chemical structure of the compound represented by various formulae, the compound represented by the formula encompasses all of a variety of single stereoisomers and mixtures of the stereoisomers in any proportions, unless otherwise specifies the configuration. Therefore, compounds represented by the formula for which the configuration is not especially specified may be a single stereoisomer or a mixture of various stereoisomers in any proportions.

In the specification, the component (A)is a compound represented by the following formula (3):

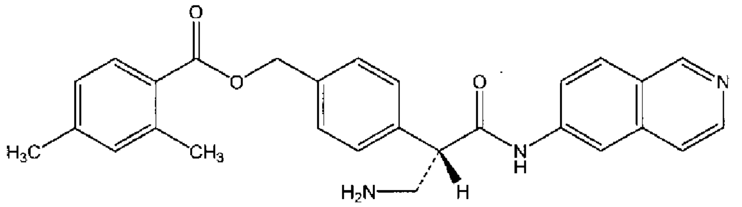

(3)

(netarsudil)(Chemical name: [4-[(2S)-3-amino-1-(isoquinolin-6-ylamino)-1-oxopropan-2-yl]phenyl]methyl 2,4-dimethylbenzoate), International Nonproprietary Name: netarsudil) or a salt thereof or a solvate thereof; and preferably a compound represented by the following formula (4) :

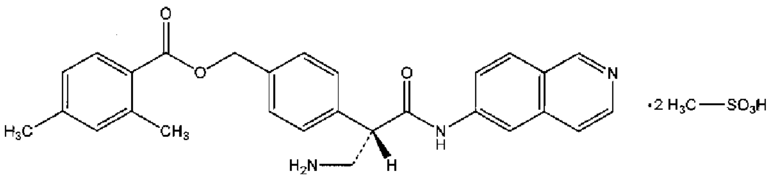

(4)

, which is a dimethanesulfonate of the compound represented by the formula (3), (chemical name: [4-[(2S)-3-amino-1-(isoquinolin-6-ylamino)-1-oxopropan-2-yl]phenyl]methyl 2,4-dimethylbenzoate dimethanesulfonate), hereinafter in the specification, it is sometimes referred to as "netarsudil dimesylate").

Component (A) is a known compound, and can be prepared by a known method. Specifically, for example, the compound can be prepared with reference to the methods described in Patent Literatures 1 to 3 or Non Patent Literature 2.

Component (A) or a salt thereof or a solvate thereof is commercially available, and these commercial products may be used. Specific examples of the commercial products include netarsudil dimesylate (the compound represented by the formula (4)) manufactured by MedChemExpress and by Chemscene LLS; and netarsudil monohydrochloride (monohydrochloride of the compound represented by the formula (3)) manufactured by Shanghai Biopharmaleader Co., Ltd.

The content of Component (A) or a salt thereof or a solvate thereof in the aqueous composition is not particularly limited, and can be appropriately determined depending on the applicable disease and patient's sex, age, or symptoms, but, from the viewpoint of obtaining an excellent pharmacological action, the content is preferably from 0.0001 to 5 w/v%, more preferably from 0.001 to 3 w/v%, still more preferably from 0.005 to 2 w/v %, and particularly preferably from 0.01 to 1 w/v% of component (A) in terms of a free form relative to the total volume of the aqueous composition.

Particularly, when netarsudil is used, the content is preferably from 0.0001 to 1 w/v%, more preferably from 0.001 to 0.5 w/v%, still more preferably from 0.005 to 0.1 w/v%, and particularly preferably from 0.01 to 0.04 w/v% of netarsudil in terms of a free form relative to the total volume of the aqueous composition, from the viewpoint of obtaining excellent pharmacological action.

<Component (B)>

The aqueous composition used in the present invention contains (B) latanoprost, in addition to the above component (A).

As specifically shown in Test Examples 1 to 6 described later, when the aqueous composition containing the component (A) is housed in a container formed of a polyolefin resin, the content of the component (A) is reduced after storage at high temperature, which is presumably caused by adsorption to the resin. As a result, it was revealed that the content reduction is suppressed by adding the component (B) to the aqueous composition.

Latanoprost (chemical name: (+)-Isopropyl (Z)-7-[(1R,2R,3R,5S)-3,5-dihydroxy-2-[(3R)-3-hydroxy-5-phenylpentyl]cyclopentyl]-5-heptenoate) and its pharmaceutically acceptable salts, as well as solvates is known, and may

be produced by a known method, or commercially available products may be used.

The content of the prostaglandin latanoprost in the aqueous composition is not particularly limited and may be appropriately determined, but, from the viewpoint of suppressing the content reduction of the component (A), it is preferably from 0.00005 to 3 w/v%, more preferably from 0.00025 to 0.25 w/v%, and particularly preferably from 0.00075 to 0.075 w/v% relative to the total volume of the aqueous composition. Above all, the content of the same is preferably from 0.0001 to 0.1 w/v%, more preferably from 0.0005 to 0.05 w/v%, and particularly preferably from 0.001 to 0.01 w/v% relative to the total volume of the aqueous composition in terms of a free form of latanoprost, from the viewpoint of suppressing the content reduction of the component (A).

The content mass ratio between the component (A) or a salt thereof or a solvate thereof and latanoprost in the aqueous composition is not particularly limited, but, from the viewpoint of suppressing the content reduction of the component (A), the content of latanoprost is preferably from 0.0001 to 50 parts by mass, more preferably from 0.0125 to 12.5 parts by mass, and particularly preferably from 0.0375 to 3.75 parts by mass in terms of a free form, relative to 1 part by mass of the component (A) or a salt thereof or a solvate thereof in terms of a free form.

In particular, the content of latanoprost is preferably from 0.005 to 5 parts by mass, more preferably from 0.025 to 2.5 parts by mass, and particularly preferably from 0.05 to 0.5 parts by mass in terms of a free form, relative to 1 part by mass of netarsudil or a salt thereof or a solvate thereof in terms of a free form, from the viewpoint of suppressing the content reduction of netarsudil.

In the present specification, the "aqueous composition" means a composition comprising at least water, and the property of the aqueous composition is not particularly limited as long as it can be housed in a container described below, and for example, the aqueous composition may be in the form of liquid (solution or suspension), or semi-solid (ointment). Examples of the water in the composition include purified water, water for injection, and sterilized purified water.

The content of water in the aqueous composition is not particularly limited, but preferably 5 % by mass or more, more preferably 20 % by mass or more, still more preferably 50 % by mass or more, even more preferably 90 % by mass or more, and particularly preferably 90 to 99.99 % by mass.

The aqueous composition may comprise one or more additives utilized in pharmaceutical products, or quasi-drugs, besides the components described above, depending on the dosage form. Examples of the additives include an inorganic salt, an isotonic agent, a chelating agent, a stabilizing agent, a pH adjusting agent, a preservative, an antioxidant, a thickening agent, a surfactant, a solubilizer, a suspending agent, a refreshing agent, a dispersing agent, a preserving agent, an oily base, an emulsion base, and a water-soluble base.

Specific examples of the additive include sodium bisulfite, benzyl benzoate, fennel oil, ethanol, an ethylene-vinyl acetate copolymer, potassium chloride, calcium chloride hydrate, sodium chloride, magnesium chloride, alkyldiaminoethylglycine hydrochloride solution, a carboxyvinyl polymer, dried sodium sulfite, dried sodium carbonate, d-camphor, dl-camphor, creatinine, chlorobutanol, geraniol, sodium chondroitin sulfate, titanium oxide, dibutylhydroxytoluene, potassium bromide, sodium hydroxide, polyoxyl 45 stearate, purified lanolin, taurine, sodium hydrogen carbonate, sodium carbonate hydrate, sodium thiosulfate hydrate, tyloxapol, sodium dehydroacetate, a concentrated mixed tocopherol, white petrolatum, peppermint water, peppermint oil, ethyl parahydroxybenzoate, butyl parahydroxybenzoate, propyl parahydroxybenzoate, methyl parahydroxybenzoate, human serum albumin, sodium pyrosulfite, phenyl ethyl alcohol, bergamot oil, benzyl alcohol, povidone, polyoxyethylene (200) polyoxypropylene glycol (70), sodium polystyrenesulfonate, polysorbate 80, polyoxyethylene hydrogenated castor oil 60, d-borneol, methanesulfonic acid, l-menthol, monoethanolamine, aluminum monostearate, polyethylene glycol monostearate, eucalyptus oil, potassium iodide, oxyquinoline sulfate, liquid paraffin, borneol, and vaseline. These may be used singly or in combination.

Among these additives, it is preferable to use potassium chloride, calcium chloride hydrate, sodium chloride, magnesium chloride, sodium hydroxide, sodium hydrogen carbonate, sodium carbonate hydrate, povidone, polysorbate 80, polyoxyethylene hydrogenated castor oil, polyethylene glycol monostearate, monoethanolamine, or l-menthol.

The aqueous composition may further comprise, in addition to the components described above, one or more other medicinal components, depending on the applicable disease. Examples of the medicinal components include $\alpha$1 receptor blockers including bunazosin or a salt thereof or a solvate thereof such as bunazosin hydrochloride; $\alpha$2 receptor agonists including brimonidine or a salt thereof or a solvate thereof such as brimonidine tartrate, and apraclonidine or a salt thereof or a solvate thereof; $\beta$-blockers including carteolol or a salt thereof or a solvate thereof such as carteolol hydrochloride, nipradilol or a salt thereof or a solvate thereof, timolol or a salt thereof or a solvate thereof such as timolol maleate, betaxolol or a salt thereof or a solvate thereof such as betaxolol hydrochloride, levobunolol or a salt thereof or a solvate thereof such as levobunolol hydrochloride, befunolol or a salt thereof or a solvate thereof, and metipranolol or a salt thereof or a solvate thereof; carbonic anhydrase inhibitors including dorzolamide or a salt thereof or a solvate thereof such as dorzolamide hydrochloride, brinzolamide or a salt thereof or a solvate thereof, acetazolamide or a salt thereof or a solvate thereof, dichlorphenamide or a salt thereof or a solvate thereof, and methazolamide or a salt thereof or a solvate thereof; Rho kinase inhibitors including Ripasudil or a salt thereof or a solvate thereof, Y-39983, and H-1129; sympathomimetic drugs including dipivefrine or a salt thereof or a solvate thereof such as dipivefrin hydrochloride,

and epinephrine or a salt thereof or a solvate thereof such as epinephrine, epinephrine borate, or epinephrine hydrochloride; parasympathomimetic drugs including distigmine bromide or a salt thereof or a solvate thereof, pilocarpine or a salt thereof or a solvate thereof such as pilocarpine, pilocarpine hydrochloride or pilocarpine nitrate, and carbachol or a salt thereof or a solvate thereof; calcium antagonists including lomerizine or a salt thereof or a solvate thereof such as lomerizine hydrochloride; and cholinesterase inhibitors including demecarium or a salt thereof or a solvate thereof, echothiophate or a salt thereof or a solvate thereof, and physostigmine or a salt thereof or a solvate thereof. These medicinal components can be blended singly or a combination of two or more.

As the other medicinal components, β-blockers are preferred, and especially timolol is preferred.

One embodiment of the aqueous compositions used in the present invention includes, for example, those other than the following <A-10>:

<A-10> a composition comprising (S)-4-(3-amino-1-(isoquinolin-6-ylamino)-1-oxopropan-2-yl)benzyl 2,4-dimethylbenzoate, latanoprost, boric acid, D-mannitol, benzalkonium chloride, polyoxyl 40 stearate, polyethylene glycol 400, EDTA, and purified water.

The pH of the aqueous composition (at 25°C) is not particularly limited, but preferably from 3 to 9, more preferably from 3.5 to 8, still more preferably from 4 to 7, particularly preferably from 5 to 6. Further, the osmotic pressure ratio of the aqueous composition to physiological saline is not particularly limited, but preferably from 0.6 to 3, particularly preferably from 0.6 to 2.

The pharmaceutical preparation of the present invention uses a container formed of a polyolefin resin, namely polypropylene.

In the present specification, a "container" means a package for directly housing the aqueous composition. The concept of the container encompasses any of "well-closed container", "tight container" and "hermetic container" which are defined in the Japanese Pharmacopoeia, Seventeenth Edition, General Notices.

The form of the container is not particularly limited as long as it is capable of housing the aqueous composition, and may be appropriately selected and set depending on the dosage form, or the application of the pharmaceutical preparation. Specific examples of the container in such a form include a container for injection, a container for inhalation, a container for spray, a bottle-shaped container, a tube-shaped container, an eye drop container, a nasal drop container, an ear drop container, or a bag shaped container.

The container is preferably an eye drop container from the viewpoint of advantageously utilizing the pharmacological action of the compound represented by the formula (1).

In the specification, the "container formed of a polyolefin resin" means a container of which at least a part in contact with the aqueous composition is "formed of a polyolefin resin". Thus, for example, a container having a polyolefin resin layer as an inner layer in contact with an aqueous composition, and further having a layer of other resin material laminated on the outside of the inner layer also corresponds to the "container formed of a polyolefin resin". The polyolefin resin is polypropylene.

It is preferred that a substance which interferes with the transmission of ultraviolet light, such as an ultraviolet absorbing agent or an ultraviolet scattering agent is kneaded in a container. With the container in which such a substance has been kneaded, the light stability of the compound represented by the formula (1) is improved. Specific examples of the ultraviolet scattering agent include titanium oxide and zinc oxide. Examples of the ultraviolet absorbing agent include: benzotriazole-based ultraviolet absorbent such as 2-(2H-benzotriazol-2-yl)-p-cresol (e.g., Tinuvin P: BASF), 2-(2H-benzotriazol-2-yl)-4,6-bis(1-methyl-1-phenylethyl)phenol (e.g., Tinuvin 234: BASF), 2-(3,5-di-tert-butyl-2-hydroxyphenyl)benzotriazole (e.g., Tinuvin 320: BASF), 2-[5-chloro-(2H)-benzotriazol-2-yl]-4-methyl-6-t-butyl-phenol (e.g., Tinuvin 326: BASF), 2-(3,5-di-t-butyl-2-hydroxyphenyl)-5-chlorobenzotriazole (e.g., Tinuvin 327: BASF), 2-(2H-benzotriazol-2-yl)-4,6-di-tert-pentylphenol (e.g., Tinuvin PA328: BASF), 2-(2H-benzotriazol-2-yl)-4-(1,1,3,3-tetramethylbutyl)phenol (e.g., Tinuvin 329: BASF), 2,2'-methylenebis [6-(2H-benzotriazol-2-yl)-4-(1,1,3,3-tetramethylbutyl)phenol (e.g., Tinuvin 360: BASF), a reaction product of methyl 3-(3-(2H-benzotriazol-2-yl)-5-tert-butyl-4-hydroxyphenyl)propionate and polyethylene glycol 300 (e.g., Tinuvin 213: BASF), 2-(2H-benzotriazol-2-yl)-6-dodecyl-4-methylphenol (e.g., Tinuvin 571: BASF), 2-(2'-hydroxy-3',5'-di-t-amylphenyl)benzotriazole, 2-[2'-hydroxy-3'-(3",4",5",6"-tetrahydrophthalimidemethyl)-5'-methylphenyl]benzotriazole, and 2,2'-methylenebis[4-(1,1,3,3-tetramethylbutyl)-6-(2H-benzotriazol-2-yl)phenol]; cyanoacrylate-based ultraviolet absorbing agent such as 2,2-bis{[2-cyano-3,3-diphenyl acryloyloxy]methyl}propane-1,3-diyl=bis(2-cyano-3,3-diphenylacrylate) (for example, Uvinul 3030 FF: BASF), ethyl 2-cyano-3,3-diphenyl acrylate (e.g., Uvinul 3035: BASF), and 2-ethylhexyl 2-cyano-3,3-diphenylacrylate (e.g., Uvinul 3039: BASF) and others; triazine-based ultraviolet absorbing agent such as 2-(4,6-diphenyl-1,3,5-triazin-2-yl)-5-[(hexyl)oxy]-phenol (e.g., Tinuvin 1577 ED: BASF); benzophenone-based ultraviolet absorbing agent such as octabenzone (e.g., Chimassorb 81: BASF), 2,2'-dihydroxy-4,4'-dimethoxy benzophenone (e.g., Uvinul 3049: BASF), 2,2'-4,4'-tetrahydrobenzophenone (e.g., Uvinul 3050: BASF), oxybenzone, hydroxymethoxybenzophenone sulfonic acid, sodium hydroxymethoxybenzophenone sulfonate, dihydroxydimethoxybenzophenone, sodium dihydroxydimethoxybenzophenone disulfonate, dihydroxybenzophenone, and tetrahydroxybenzophenone; cinnamic acid-based ultraviolet absorbing agent such as methyl diisopropylcinnamate, cinoxate, glyceryl mono-2-ethylhexanoate diparamethoxycinnamate, a mixture of isopropyl paramethoxycinnamate and diisopropyl cinnamate, 2-ethylhexyl paramethoxycinnamate, and benzyl cinnamate; benzoic acid ester-based ultraviolet absorbing agent such as para-aminobenzoic acid, ethyl para-aminobenzoate, glyceryl para-aminobenzoate, amyl para-dimethylaminobenzoate, 2-ethylhexyl para-dimethylaminobenzoate, and ethyl 4-[N,N-di(2-hydroxypropyl) amino]benzoate; salicylic acid-based ultraviolet absorbing agent such as ethylene glycol salicylate, octyl salicylate, dipropylene glycol salicylate, phenyl salicylate, homomenthyl salicylate, and methyl salicylate; guaiazulene; 2-ethylhexyl dimethoxybenzylidene dioxoimidazolidine propionate; 2,4,6-tris[4-(2-ethylhexyloxycarbonyl)anilino] 1,3,5-triazine; para-hydroxy anisole; 4-tert-butyl-4'-methoxydibenzoylmethane; phenylbenzimidazole sulfonate; and hexyl 2-(4-diethylamino-2-hydroxybenzoyl) benzoate.

When a substance which interferes with the transmission of ultraviolet light is kneaded in the container, the blending ratio is determined depending on the kinds of the substance, but, for example, the ratio of the substance in the container may be about from 0.001 to 50 % by mass, preferably from 0.002 to 25 % by mass, particularly preferably from 0.01 to 10 % by mass.

It is preferred that the inside of the container formed of polypropylene is visible (observable) to the naked eye. When the inside is visible, there are some advantages that it becomes possible to examine the presence or absence of contamination in the manufacturing process of the pharmaceutical preparation; and that the user of the pharmaceutical preparation can check the remained content (aqueous composition). Here, it is sufficient if the visibility is ensured in at least a portion of the surface of the container (For example, an eye drop container having a bottom surface allowing the inside of the container to be visible corresponds to the container which is visible, even when the inside of the container is not visible from the side surface owing to a shrink film). When the inside of the container is visible from at least a portion of the surface of the container, it is possible to confirm the aqueous composition inside of the container.

The means for housing the aqueous composition in the container formed of polypropylene is not particularly limited and it can be performed by filling with an ordinary method depending on the form of the container.

The pharmaceutical preparation or aqueous composition can be formulated in various dosage forms according to a known method described in the Japanese Pharmacopoeia, Seventeenth Edition, General Rules for Preparation. Examples of the dosage form include injection, inhalation liquid, eye drop, eye ointment, ear drop, nasal drop liquid, enema formulation, topical liquid, spray, ointment, gel, oral liquid, and syrup. From the viewpoint of advantageously utilizing the pharmacological actions of the component (A), the dosage form is preferably, a dosage form for eye disease, specifically, eye drop or eye ointment, and particularly preferably, eye drop.

The applicable disease of the pharmaceutical preparation is not particularly limited, and it is appropriately selected depending on the pharmacological actions of the component (A).

Specifically, for example, the pharmaceutical preparation can be used as a prevention or treatment agent for ocular hypertension and glaucoma, based on Rho kinase inhibitory activity, norepinephrine transporter inhibitory activity, and intraocular pressure-decreasing activity of the component (A). Detailed examples of the glaucoma include primary open-angle glaucoma, normal tension glaucoma, hypersecretion glaucoma, acute angle closure glaucoma, chronic angle closure glaucoma, plateau iris syndrome, mixed-type glaucoma, steroid glaucoma, glaucoma capsulare, pigment glaucoma, amyloid glaucoma, angiogenesis glaucoma, and malignant glaucoma.

In the case of using the aqueous composition or pharmaceutical preparation as a prevention or treatment agent for eye disease (appropriately, a disease selected from the group of ocular hypertension and glaucoma), the aqueous composition or pharmaceutical preparation may be administered, for example, about 1 to 3 times a day in a suitable dose.

## EXAMPLES:

Next, the present invention is further described by way of Examples, but the present invention is not limited to these Examples.

In the following Test Examples, the measurements of netarsudil using HPLC were performed using an ODS column as the column, 0.01 mol/L phosphate buffer solution and acetonitrile as the mobile phase, and a UV absorptiometer as the detector (wavelength: 254 nm), respectively.

### [Test Example 1] Adsorption Inhibition Test 1

To investigate the presence or absence of adsorption of netarsudil to the container formed of a polyolefin resin, a piece formed of a polyolefin resin was placed in an aqueous composition comprising netarsudil and stored for a certain period of time and then the presence or absence of the content reduction of netarsudil in the aqueous composition was examined.

In other words, aqueous compositions shown in Table 1 were prepared with an ordinary method, and 5 mL of the obtained composition each was housed in a glass container, and then, into the aqueous composition, three resin pieces (each having a size of 1 cm × 2 cm) formed of polypropylene (PP) were immersed to obtain a pharmaceutical preparation of Reference Example 1 or 2. In addition, a pharmaceutical preparation of Reference Example 3 was prepared

in the same way as Reference Example 2 except for not immersing resin pieces formed of polypropylene.

Each of the obtained pharmaceutical preparations was stored at 60°C for a week. To examine the presence or absence of content reduction of netarsudil after storage, the concentrations of netarsudil in the aqueous composition before and after storage were measured for each of the pharmaceutical preparations. The concentration of netarsudil in the aqueous composition was calculated by measuring the ratio of the peak area of the aqueous composition relative to the peak area of a netarsudil solution of a known concentration, using HPLC.

Then, based on the calculated concentration of netarsudil in the aqueous composition, the residual rate (%) of netarsudil was assessed using the following expression:

Residual rate (%) = {(Concentration of netarsudil in the aqueous composition after storage)/(Concentration of netarsudil in the aqueous composition before storage)} × 100

The results are shown in Table 1.

[Table 1]

| | | Reference Example 1 | Reference Example 2 | Reference Example 3 |
|---|---|---|---|---|
| Aqueous composition (amount: per 100 mL) | Netarsudil dimesylate | 0.02848 g (0.02 g in terms of a free form) | 0.02848 g (0.02 g in terms of a free form) | 0.02848 g (0.02 g in terms of a free form) |
| | Boric acid | 0.05 g | - | - |
| | Sodium hydroxide | q.s. (pH 5.0) | q.s. (pH 5.0) | q.s. (pH 5.0) |
| | Purified water | Balance | Balance | Balance |
| Presence or absence of immersion of resin piece formed of PP | | Presence | Presence | Absence |
| Residual rate (%) | | 84.5 | 74.9 | \| 83.1 |

As shown in the results listed in Table 1, from the comparison between Reference Examples 2 and 3, the content of netarsudil in the aqueous composition after stored under high temperature conditions was further reduced due to the immersion of the pieces formed of polypropylene. Such a content reduction was caused by the immersion of the pieces formed of polypropylene, so that it is assumed that netarsudil was adsorbed to the pieces formed of polypropylene.

Meanwhile, from the comparison between Reference Examples 1 and 2, it was revealed that the content reduction of netarsudil was suppressed when the aqueous composition comprising netarsudil further comprised a boric acid.

From the test results above, it was revealed that if the aqueous composition comprising the compound represented by the formula (1) exemplified by netarsudil or a salt thereof or a solvate thereof is further comprised of an acid such as borate exemplified by a boric acid, the content reduction (adsorption) which may occur in the aqueous composition housed in a container formed of a polyolefin resin exemplified by polypropylene after storage under high temperatures can be relatively suppressed.

[Test Example 2] Adsorption Inhibition Test 2

The procedures of Test Example 1 were repeated with the exception that the formulation of the aqueous composition was changed as shown in Table 2, thereby performing this test.

The results are shown in Table 2.

[Table 2]

| | | Reference Example 4 | Reference Example 5 | Reference Example 6 |
|---|---|---|---|---|
| Aqueous composition (amount: per 100 mL) | Netarsudil dimesylate | 0.02848 g (0.02 g in terms of a free form) | 0.02848 g (0.02 g in terms of a free form) | 0.02848 g (0.02 g in terms of a free form) |
| | Disodium edetate hydrate | 0.01 g | - | - |
| | Sodium hydroxide | q.s. (pH 5.0) | q.s. (pH 5.0) | q.s. (pH 5.0) |
| | Purified water | Balance | Balance | Balance |
| Presence or absence of immersion of resin piece formed of PP | | Presence | Presence | Absence |
| Residual rate (%) | | 83.9 | 74.9 | \| 83.1 |

As shown in the results listed in Table 2, from the comparison between Reference Examples 4 and 5, it was revealed that the content reduction of netarsudil was suppressed when the aqueous composition comprising netarsudil further comprised disodium edetate hydrate.

From the test results above, it was revealed that if the aqueous composition comprising the compound represented by the formula (1) exemplified by netarsudil or a salt thereof or a solvate thereof is further comprised of an acid such as aliphatic carboxylate exemplified by disodium edetate hydrate, the content reduction (adsorption) which may occur in the aqueous composition housed in a container formed of a polyolefin resin exemplified by polypropylene after storage under high temperatures can be relatively suppressed.

[Test Example 3] Adsorption Inhibition Test 3

The procedures of Test Example 1 were repeated with the exception that the formulation of the aqueous composition was changed as shown in Table 3, thereby performing this test.

The results are shown in Table 3.

[Table 3]

| | | Reference Example 7 | Reference Example 8 | Reference Example 9 |
|---|---|---|---|---|
| Aqueous composition (amount: per 100 mL) | Netarsudil dimesylate | 0.02848 g (0.02 g in terms of a free form) | 0.02848 g (0.02 g in terms of a free form) | 0.02848 g (0.02 g in terms of a free form) |
| | Benzalkonium chloride | 0.015 g | - | - |
| | Sodium hydroxide | q.s. (pH 5.0) | q.s. (pH 5.0) | q.s. (pH 5.0) |
| | Purified water | Balance | Balance | Balance |
| Presence or absence of immersion of resin piece formed of PP | | Presence | Presence | Absence |
| Residual rate (%) | | 85.4 | 74.9 | \| 83.1 |

As shown in the results listed in Table 3, from the comparison between Reference Examples 7 and 8, it was revealed that the content reduction of netarsudil was suppressed when the aqueous composition comprising netarsudil further comprised benzalkonium chloride.

From the test results above, it was revealed that if the aqueous composition comprising the compound represented by the formula (1) exemplified by netarsudil or a salt thereof or a solvate thereof is further comprised of a quaternary ammonium type surfactant exemplified by benzalkonium chloride, the content reduction (adsorption) which may occur in the aqueous composition housed in a container formed of a polyolefin resin exemplified by polypropylene after storage under high temperatures can be relatively suppressed.

[Test Example 4] Adsorption Inhibition Test 4

The procedures of Test Example 1 were repeated with the exception that the formulation of the aqueous composition was changed as shown in Table 4, thereby performing this test.
The results are shown in Table 4.

[Table 4]

| | | Reference Example 10 | Reference Example 11 | Reference Example 12 |
|---|---|---|---|---|
| Aqueous composition (amount: per 100 mL) | Netarsudil dimesylate | 0.02848 g (0.02 g in terms of a free form) | 0.02848 g (0.02 g in terms of a free form) | 0.02848 g (0.02 g in terms of a free form) |
| | D-mannitol | 4.7 g | - | - |
| | Sodium hydroxide | q.s. (pH 5.0) | q.s. (pH 5.0) | q.s. (pH 5.0) |
| | Purified water | Balance | Balance | Balance |
| Presence or absence of immersion of resin piece formed of PP | | Presence | Presence | Absence |
| Residual rate (%) | | 88.1 | 74.9 | \| 83.1 |

As shown in the results listed in Table 4, from the comparison between Reference Examples 10 and 11, it was revealed that the content reduction of netarsudil was suppressed when the aqueous composition comprising netarsudil further comprised D-mannitol.
From the test results above, it was revealed that if the aqueous composition comprising the compound represented by the formula (1) exemplified by netarsudil or a salt thereof or a solvate thereof is further comprised of a polyhydric alcohol exemplified by D-mannitol, the content reduction (adsorption) which may occur in the aqueous composition housed in a container formed of a polyolefin resin exemplified by polypropylene after storage under high temperatures can be relatively suppressed.

[Test Example 5] Adsorption Inhibition Test 5

The procedures of Test Example 1 were repeated with the exception that the formulation of the aqueous composition was changed as shown in Table 5, thereby performing this test.
The results are shown in Table 5.

[Table 5]

| | | Reference Example 13 | Reference Example 14 | Reference Example 15 |
|---|---|---|---|---|
| Aqueous composition (amount: per 100 mL) | Netarsudil dimesylate | 0.02848 g (0.02 g in terms of a free form) | 0.02848 g (0.02 g in terms of a free form) | 0.02848 g (0.02 g in terms of a free form) |
| | Macrogol 400 | 2.5 g | - | - |
| | Sodium hydroxide | q.s. (pH 5.0) | q.s. (pH 5.0) | q.s. (pH 5.0) |
| | Purified water | Balance | Balance | Balance |
| Presence or absence of immersion of resin piece formed of PP | | Presence | Presence | Absence |
| Residual rate (%) | | 88.9 | 74.9 | \| 83.1 |

As shown in the results listed in Table 5, from the comparison between Reference Examples 13 and 14, it was revealed that the content reduction of netarsudil was suppressed when the aqueous composition comprising netarsudil further comprised macrogol 400.

From the test results above, it was revealed that if the aqueous composition comprising the compound represented by the formula (1) exemplified by netarsudil or a salt thereof or a solvate thereof is further comprised of a polyhydric alcohol exemplified by macrogol 400, the content reduction (adsorption) which may occur in the aqueous composition housed in a container formed of a polyolefin resin exemplified by polypropylene after storage under high temperatures can be relatively suppressed.

[Test Example 6] Adsorption Inhibition Test 6

The procedures of Test Example 1 were repeated with the exception that the formulation of the aqueous composition was changed as shown in Table 6, thereby performing this test.
The results are shown in Table 6.

[Table 6]

| | | Reference Example 16 | Reference Example 17 | Reference Example 18 |
|---|---|---|---|---|
| Aqueous composition (amount: per 100 mL) | Netarsudil dimesylate | 0.02848 g (0.02 g in terms of a free form) | 0.02848 g (0.02 g in terms of a free form) | 0.02848 g (0.02 g in terms of a free form) |
| | Latanoprost | 0.005 g | - | - |
| | Sodium hydroxide | q.s. (pH 5.0) | q.s. (pH 5.0) | q.s. (pH 5.0) |
| | Purified water | Balance | Balance | Balance |
| Presence or absence of immersion of resin piece formed of PP | | Presence | Presence | Absence |
| Residual rate (%) | | 87.4 | 74.9 | 83.1 |

As shown in the results listed in Table 6, from the comparison between Reference Examples 16 and 17, it was revealed that the content reduction of netarsudil was suppressed when the aqueous composition comprising netarsudil further comprised latanoprost.
From the test results above, it was revealed that if the aqueous composition comprising the compound represented by the formula (1) exemplified by netarsudil or a salt thereof or a solvate thereof is further comprised of a prostaglandin compound exemplified by latanoprost, the content reduction (adsorption) which may occur in the aqueous composition housed in a container formed of a polyolefin resin exemplified by polypropylene after storage under high temperatures can be relatively suppressed.

[Test Example 7] Heat Stability Test

The heat stability of netarsudil in the aqueous composition was assessed by examining the presence or absence of content reduction of netarsudil after stored under high temperature for a certain period.
In other words, an aqueous composition having the formulation shown in Table 7 was prepared with an ordinary method, and then the obtained composition was housed in a container formed of low-density polyethylene (LDPE), high density-polyethylene (HDPE) or polypropylene (PP) to obtain pharmaceutical preparations. In addition, the same aqueous composition was housed in a container formed of glass to obtain a pharmaceutical preparation of Comparative Example.
Each of the obtained pharmaceutical preparations was stored at 80°C for a week. To examine the presence or absence of content reduction of netarsudil after storage, the concentrations of netarsudil in the aqueous composition before and after storage were measured according to the same method as in Test Example 1 for each of the pharmaceutical preparation.
Then, based on the calculated concentration of netarsudil in the aqueous composition, the residual rate (%) of netarsudil was assessed using the following expression:

Residual rate (%) = {(Concentration of netarsudil in the aqueous composition after storage)/(Concentration of netarsudil in the aqueous composition before storage)} × 100

The results are shown in Table 8.

[Table 7]

| Component | Amount (per 100 mL) |
|---|---|
| Netarsudil dimesylate | 0.02848 g (0.02 g in terms of a free form) |
| Boric acid | 0.05 g |
| Sodium hydroxide | q.s. (pH 5.0) |
| Purified water | Balance |

Table 8]

| | Material of container | After storage at 80°C for a week |
|---|---|---|
| Residual rate (%) | LDPE | 72.1 |
| | HDPE | 71.4 |
| | PP | 70.7 |
| | Comparative Example: glass | 47.5 |

As shown in the results listed in Table 8, when the aqueous composition comprising netarsudil was housed in the container formed of polyethylene (LDPE, HDPE) or the container formed of polypropylene (PP), the content reduction due to storage under high temperature was suppressed relative to the case housed in a container formed of glass (Comparative Example).

From the results of Test Example 7, it was revealed that, by housing the aqueous composition comprising the compound represented by the formula (1) exemplified by netarsudil or a salt thereof or a solvate thereof in a container formed of a polyolefin resin, the content reduction due to storage under high temperature was relatively suppressed and a pharmaceutical preparation having excellent storage stability was obtained.

[Preparation Examples 1 to 36] (Comparative)

Pharmaceutical preparations of Preparation Examples 1 to 36 can be prepared by preparing aqueous compositions each comprising the components in amounts ((g) per 100 mL of the aqueous composition) shown in Tables 9 to 17 with an ordinary method, and then housing the prepared aqueous composition each in an eye drop container formed of high-density polyethylene.

[Table 9]

| | Preparation Examples | | | |
|---|---|---|---|---|
| | 1 | 2 | 3 | 4 |
| Netarsudil dimesylate | 0.02848 (0.02 in terms of a free form) | 0.02848 (0.02 in terms of a free form) | 0.02848 (0.02 in terms of a free form) | 0.02848 (0.02 in terms of a free form) |
| Boric acid | 0.05 | 0.3 | | |
| Borax | | 0.3 | | |

(continued)

| | Preparation Examples | | | |
|---|---|---|---|---|
| | 1 | 2 | 3 | 4 |
| Sodium dihydrogen phosphate | | | 0.031 | 0.5 |
| Disodium hydrogen phosphate | | | 0.07 | 0.8 |
| Citric acid | | | | |
| Sodium citrate | | | | |
| Acetic acid | | | | |
| Sodium acetate | | | | |
| Disodium edetate | | 0.01 | 0.01 | |
| D-mannitol | 4.7 | | | |
| Glycerin | | 2.5 | | |
| Propylene glycol | | | 1.7 | |
| Xylitol | | | 0.7 | |
| Macrogol 6000 | | | | 1 |
| Macrogol 4000 | | | | 4 |
| Sorbitol | | | | |
| Trometamol | | | | |
| Benzalkonium chloride | 0.015 | 0.03 | 0.05 | |
| Benzododecinium bromide | | | | 0.01 |
| Benzethonium chloride | | | | 0.01 |
| Sodium chloride | | 0.1 | | |
| Potassium chloride | | | | 0.2 |
| Calcium chloride | | | | |
| Sodium hydroxide | q.s. | q.s. | q.s. | q.s. |
| Hydrochloric acid | q.s. | q.s. | q.s. | q.s. |
| Purified water | Total Amount 100 mL | Total Amount 100 mL | Total Amount 100 mL | Total Amount 100 mL |
| pH | 5.0 | 5.5 | 6.0 | 5.0 |

[Table 10]

| | Preparation Examples | | | |
|---|---|---|---|---|
| | 5 | 6 | 7 | 8 |
| Netarsudil dimesylate | 0.02848 (0.02 in terms of a free form) | 0.02848 (0.02 in terms of a free form) | 0.02848 (0.02 in terms of a free form) | 0.02848 (0.02 in terms of a free form) |
| Boric acid | | | | |
| Borax | | | | |
| Sodium dihydrogen phosphate | | | | |
| Disodium hydrogen phosphate | | | | |
| Citric acid | 0.12 | 0.05 | | |
| Sodium citrate | 0.4 | 0.03 | | |
| Acetic acid | | | 0.001 | |
| Sodium acetate | | | 0.2 | 0.1 |
| Disodium edetate | | 0.01 | 0.01 | |
| D-mannitol | | | 1 | 3 |
| Glycerin | | | 3 | |
| Propylene glycol | | | | 1 |
| Xylitol | | | | |
| Macrogol 6000 | | | | |
| Macrogol 4000 | | | | |
| Sorbitol | 0.86 | | | |
| Trometamol | | 20 | | |
| Benzalkonium chloride | 0.015 | 0.03 | 0.05 | |
| Benzododecinium bromide | | | | 0.01 |
| Benzethonium chloride | | | | 0.01 |
| Sodium chloride | | | 0.05 | |
| Potassium chloride | | | 0.05 | |
| Calcium chloride | 0.1 | | | |
| Sodium hydroxide | q.s. | q.s. | q.s. | q.s. |
| Hydrochloric acid | q.s. | q.s. | q.s. | q.s. |
| Purified water | Total Amount 100 mL | Total Amount 100 mL | Total Amount 100 mL | Total Amount 100 mL |
| pH | 5.0 | 5.5 | 6.0 | 5.0 |

[Table 11]

| | Preparation Examples | | | |
|---|---|---|---|---|
| | 9 | 10 | 11 | 12 |
| Netarsudil dimesylate | 0.01424 (0.01 in terms of a free form | 0.05696 (0.04 in terms of a free form) | 0.02848 (0.02 in terms of a free form) | 0.02848 (0.02 in terms of a free form) |
| Boric acid | 0.05 | 0.3 | | |
| Borax | | 0.3 | | |
| Sodium dihydrogen phosphate | | | 0.031 | 0.5 |
| Disodium hydrogen phosphate | | | 0.07 | 0.8 |
| Citric acid | | | | |
| Sodium citrate | | | | |
| Acetic acid | | | | |
| Sodium acetate | | | | |
| Disodium edetate | | 0.01 | 0.01 | |
| D-mannitol | 2 | 2 | | 2 |
| Glycerin | 2 | 2 | 1 | |
| Propylene glycol | | | | 1.5 |
| Xylitol | 0.3 | | | |
| Macrogol 6000 | 1 | | | |
| Macrogol 4000 | | 2 | | |
| Sorbitol | | | 0.5 | |
| Trometamol | | | 10 | |
| Benzalkonium chloride | 0.015 | 0.03 | 0.05 | |
| Benzododecinium bromide | | | | 0.01 |
| Benzethonium chloride | | | | 0.01 |
| Sodium chloride | | 0.1 | | |
| Potassium chloride | | | | 0.2 |
| Calcium chloride | | | | |
| Sodium hydroxide | q.s. | q.s. | q.s. | q.s. |
| Hydrochloric acid | q.s. | q.s. | q.s. | q.s. |
| Purified water | Total Amount 100 mL | Total Amount 100 mL | Total Amount 100 mL | Total Amount 100 mL |
| pH | 5.0 | 5.5 | 6.0 | 5.0 |

[Table 12]

| | Preparation Examples | | | |
|---|---|---|---|---|
| | 13 | 14 | 15 | 16 |
| Netarsudil dimesylate | 0.02848 (0.02 in terms of a free form) | 0.02848 (0.02 in terms of a free form) | 0.01424 (0.01 in terms of a free form) | 0.05696 (0.04 in terms of a free form) |
| Boric acid | | | | |
| Borax | | | | |
| Sodium dihydrogen phosphate | | | | |
| Disodium hydrogen phosphate | | | | |
| Citric acid | 0.12 | 0.05 | | |
| Sodium citrate | 0.4 | 0.03 | | |
| Acetic acid | | | 0.001 | |
| Sodium acetate | | | 0.2 | 0.1 |
| Disodium edetate | | 0.01 | 0.01 | |
| D-mannitol | 3 | 1 | 1 | 2 |
| Glycerin | 1 | | 1 | |
| Propylene glycol | | | | 1 |
| Xylitol | | | | |
| Macrogol 6000 | | 1 | | |
| Macrogol 4000 | | 2 | | |
| Sorbitol | | | | 0.5 |
| Trometamol | | | 15 | |
| Benzalkonium chloride | 0.015 | 0.03 | 0.05 | |
| Benzododecinium bromide | | | | 0.01 |
| Benzethonium chloride | | | | 0.01 |
| Sodium chloride | | | 0.05 | |
| Potassium chloride | | | 0.05 | |
| Calcium chloride | 0.1 | | | |
| Sodium hydroxide | q.s. | q.s. | q.s. | q.s. |
| Hydrochloric acid | q.s. | q.s. | q.s. | q.s. |
| Purified water | Total Amount 100 mL | Total Amount 100 mL | Total Amount 100 mL | Total Amount 100 mL |
| pH | 5.0 | 5.5 | 6.0 | 5.0 |

[Table 13]

| | Preparation Examples | | | |
|---|---|---|---|---|
| | 17 | 18 | 19 | 20 |
| Netarsudil dimesylate | 0.02848 (0.02 in terms of a free form) | 0.02848 (0.02 in terms of a free form) | 0.02848 (0.02 in terms of a free form) | 0.02848 (0.02 in terms of a free form) |
| Latanoprost | 0.005 | | | |
| Travoprost | | 0.004 | | |
| Bimatoprost | | | 0.3 | |
| Tafluprost | | | | 0.0015 |
| Boric acid | 0.05 | 0.3 | | |
| Borax | | 0.3 | | |
| Sodium dihydrogen phosphate | | | 0.031 | 0.5 |
| Disodium hydrogen phosphate | | | 0.07 | 0.8 |
| Citric acid | | | | |
| Sodium citrate | | | | |
| Acetic acid | | | | |
| Sodium acetate | | | | |
| Disodium edetate | 0.01 | 0.01 | 0.01 | 0.01 |
| D-mannitol | 4.7 | | | |
| Glycerin | | 2.5 | | |
| Propylene glycol | | | 1.7 | |
| Xylitol | | | 0.7 | |
| Macrogol 400 | 2.5 | 1 | | 4 |
| Macrogol 4000 | | | | 1 |
| Sorbitol | | | | |
| Trometamol | | | | |
| Benzalkonium chloride | 0.2 | 0.03 | 0.05 | |
| Benzododecinium bromide | | | | 0.01 |
| Benzethonium chloride | | | | 0.01 |
| Polyoxyl 40 stearate (Myrj-52) | 0.5 | | | |
| Cremophor RH40 | | 0.5 | | |
| Polyoxyethylene hydrogenated castor oil 40 | | | 0.5 | |
| Polysorbate 80 | | | | 0.5 |
| Sodium hydroxide | q.s. | q.s. | q.s. | q.s. |
| Hydrochloric acid | q.s. | q.s. | q.s. | q.s. |

(continued)

| | Preparation Examples | | | |
|---|---|---|---|---|
| | 17 | 18 | 19 | 20 |
| Netarsudil dimesylate | 0.02848 (0.02 in terms of a free form) | 0.02848 (0.02 in terms of a free form) | 0.02848 (0.02 in terms of a free form) | 0.02848 (0.02 in terms of a free form) |
| Purified water | Total Amount 100 mL | Total Amount 100 mL | Total Amount 100 mL | Total Amount 100 mL |
| pH | 5.0 | 5.0 | 5.0 | 5.0 |

[Table 14]

| | Preparation Examples | | | |
|---|---|---|---|---|
| | 21 | 22 | 23 | 24 |
| Netarsudil dimesylate | 0.02848 (0.02 in terms of a free form) | 0.02848 (0.02 in terms of a free form) | 0.02848 (0.02 in terms of a free form) | 0.02848 (0.02 in terms of a free form) |
| Latanoprost | 0.005 | | | |
| Travoprost | | 0.004 | | |
| Bimatoprost | | | 0.3 | |
| Tafluprost | | | | 0.0015 |
| Boric acid | | | | |
| Borax | | | | |
| Sodium dihydrogen phosphate | | | | |
| Disodium hydrogen phosphate | | | | |
| Citric acid | 0.12 | 0.05 | | |
| Sodium citrate | 0.4 | 0.03 | | |
| Acetic acid | | | 0.001 | |
| Sodium acetate | | | 0.2 | 0.1 |
| Disodium edetate | | 0.01 | 0.01 | |
| D-mannitol | | | 1 | 3 |
| Glycerin | | | 3 | |
| Propylene glycol | | | | 1 |
| Xylitol | | | | |
| Macrogol 400 | | | | |
| Macrogol 4000 | | | | |
| Sorbitol | 0.86 | | | |
| Trometamol | | 20 | | |
| Benzalkonium chloride | 0.015 | 0.03 | 0.05 | |
| Benzododecinium bromide | | | | 0.01 |
| Benzethonium chloride | | | | 0.01 |

(continued)

| | Preparation Examples | | | |
|---|---|---|---|---|
| | 21 | 22 | 23 | 24 |
| Polyoxyl 40 stearate (Myrj-52) | 0.5 | | | |
| Cremophor RH40 | | 0.5 | | |
| Polyoxyethylene hydrogenated castor oil 40 | | | 0.5 | |
| Polysorbate 80 | | | | 0.5 |
| Sodium hydroxide | q.s. | q.s. | q.s. | q.s. |
| Hydrochloric acid | q.s. | q.s. | q.s. | q.s. |
| Purified water | Total Amount 100 mL | Total Amount 100 mL | Total Amount 100 mL | Total Amount 100 mL |
| pH | 6.0 | 5.5 | 6.0 | 5.5 |

[Table 15]

| | Preparation Examples | | | |
|---|---|---|---|---|
| | 25 | 26 | 27 | 28 |
| Netarsudil dimesylate | 0.02848 (0.02 in terms of a free form) | 0.02848 (0.02 in terms of a free form) | 0.02848 (0.02 in terms of a free form) | 0.02848 (0.02 in terms of a free form) |
| Latanoprost | 0.005 | | | |
| Travoprost | | 0.004 | | |
| Bimatoprost | | | 0.3 | |
| Tafluprost | | | | 0.0015 |
| Boric acid | 0.05 | 0.3 | | |
| Borax | | 0.3 | | |
| Sodium dihydrogen phosphate | | | 0.031 | 0.5 |
| Disodium hydrogen phosphate | | | 0.07 | 0.8 |
| Citric acid | | | | |
| Sodium citrate | | | | |
| Acetic acid | | | | |
| Sodium acetate | | | | |
| Disodium edetate | | 0.01 | 0.01 | |
| D-mannitol | 2 | 2 | | 2 |
| Glycerin | 2 | 2 | 1 | |
| Propylene glycol | | | | 1.5 |
| Xylitol | 0.3 | | | |
| Macrogol 400 | | 2 | | |
| Macrogol 4000 | | 1 | | |

(continued)

| | Preparation Examples | | | |
|---|---|---|---|---|
| | 25 | 26 | 27 | 28 |
| Sorbitol | | | 0.5 | |
| Trometamol | | | 10 | |
| Benzalkonium chloride | 0.015 | 0.03 | 0.05 | |
| Benzododecinium bromide | | | | 0.01 |
| Benzethonium chloride | | | | 0.01 |
| Polyoxyl 40 stearate (Myrj-52) | 0.5 | | | |
| Cremophor RH40 | | 0.5 | | |
| Polyoxyethylene hydrogenated castor oil 40 | | | 0.5 | |
| Polysorbate 80 | | | | 0.5 |
| Sodium hydroxide | q.s. | q.s. | q.s. | q.s. |
| Hydrochloric acid | q.s. | q.s. | q.s. | q.s. |
| Purified water | Total Amount 100 mL | Total Amount 100 mL | Total Amount 100 mL | Total Amount 100 mL |
| pH | 7.0 | 6.0 | 7.0 | 6.0 |

[Table 16]

| | Preparation Examples | | | |
|---|---|---|---|---|
| | 29 | 30 | 31 | 32 |
| Netarsudil dimesylate | 0.02848 (0.02 in terms of a free form) | 0.02848 (0.02 in terms of a free form) | 0.02848 (0.02 in terms of a free form) | 0.02848 (0.02 in terms of a free form) |
| Latanoprost | 0.005 | | | |
| Travoprost | | 0.004 | | |
| Bimatoprost | | | 0.3 | |
| Tafluprost | | | | 0.0015 |
| Boric acid | | | | |
| Borax | | | | |
| Sodium dihydrogen phosphate | | | | |
| Disodium hydrogen phosphate | | | | |
| Citric acid | 0.12 | 0.05 | | |
| Sodium citrate | 0.4 | 0.03 | | |
| Acetic acid | | | 0.001 | |
| Sodium acetate | | | 0.2 | 0.1 |
| Disodium edetate | | 0.01 | 0.01 | |

(continued)

| | Preparation Examples | | | |
|---|---|---|---|---|
| | 29 | 30 | 31 | 32 |
| D-mannitol | 3 | 1 | 1 | 2 |
| Glycerin | 1 | | 1 | |
| Propylene glycol | | | | 1 |
| Xylitol | | | | |
| Macrogol 400 | | 2 | | |
| Macrogol 4000 | | 1 | | |
| Sorbitol | | | | 0.5 |
| Trometamol | | | 15 | |
| Benzalkonium chloride | 0.015 | 0.03 | 0.05 | |
| Benzododecinium bromide | | | | 0.01 |
| Benzethonium chloride | | | | 0.01 |
| Polyoxyl 40 stearate (Myrj-52) | 0.5 | | | |
| Cremophor RH40 | | 0.5 | | |
| Polyoxyethylene hydrogenated castor oil 40 | | | 0.5 | |
| Polysorbate 80 | | | | 0.5 |
| Sodium hydroxide | q.s. | q.s. | q.s. | q.s. |
| Hydrochloric acid | q.s. | q.s. | q.s. | q.s. |
| Purified water | Total Amount 100 mL | Total Amount 100 mL | Total Amount 100 mL | Total Amount 100 mL |
| pH | 6.0 | 5.5 | 6.0 | 5.5 |

[Table 17]

| | Preparation Examples | | | |
|---|---|---|---|---|
| | 33 | 34 | 35 | 36 |
| Netarsudil dimesylate | 0.02848 (0.02 in terms of a free form) | 0.02848 (0.02 in terms of a free form) | 0.02848 (0.02 in terms of a free form) | 0.02848 (0.02 in terms of a free form) |
| Timolol maleate | 0.683 (0.5 mg in terms of a free form) | 0.683 (0.5 mg in terms of a free form) | 0.683 (0.5 mg in terms of a free form) | 0.683 (0.5 mg in terms of a free form) |
| Boric acid | 0.04 | 0.05 | | |
| Borax | 0.01 | | | |
| Sodium dihydrogen phosphate | | 0.031 | | |
| Disodium hydrogen phosphate | | 0.07 | | |
| Citric acid | | | 0.05 | |

(continued)

| | Preparation Examples | | | |
|---|---|---|---|---|
| | 33 | 34 | 35 | 36 |
| Sodium citrate | | | 0.03 | |
| Acetic acid | | | | 0.001 |
| Sodium acetate | | | | 0.2 |
| Disodium edetate | 0.01 | 0.01 | 0.01 | 0.01 |
| D-mannitol | 4.7 | | | |
| Glycerin | | 2.5 | | |
| Propylene glycol | | | 1.7 | |
| Xylitol | | | 0.7 | |
| Macrogol 400 | | 1 | | |
| Macrogol 4000 | | | 1 | |
| Sorbitol | | | | 3 |
| Trometamol | | | | 15 |
| Benzalkonium chloride | 0.015 | 0.03 | 0.05 | |
| Benzododecinium bromide | | | | 0.01 |
| Benzethonium chloride | | | | 0.01 |
| Methyl cellulose | 0.5 | 2 | | |
| Gellan gum | | | 0.2 | 0.6 |
| Sodium hydroxide | q.s. | q.s. | q.s. | q.s. |
| Hydrochloric acid | q.s. | q.s. | q.s. | q.s. |
| Purified water | Total Amount 100 mL | Total Amount 100 mL | Total Amount 100 mL | Total Amount 100 mL |
| pH | 5.0 | 6.0 | 7.0 | 6.0 |

[Preparation Examples 37 to 72]

Pharmaceutical preparations of Preparation Examples 37 to 72 can be prepared in the same way as in Preparation Examples 1 to 36 except for using an eye drop container formed of polypropylene instead of that formed of high-density polyethylene.

[Preparation Examples 73 to 108] (Comparative)

Pharmaceutical preparations of Preparation Examples 73 to 108 can be prepared in the same way as in Preparation Examples 1 to 36 except for using an eye drop container formed of cyclic polyolefin instead of that formed of high-density polyethylene.

[Preparation Examples 109 to 144] (Comparative)

Pharmaceutical preparations of Preparation Examples 109 to 144 can be prepared in the same way as in Preparation Examples 1 to 36 except for using an eye drop container formed of low-density polyethylene instead of that formed of high-density polyethylene.

[Preparation Examples 145 to 288] (Comparative)

Pharmaceutical preparations of Preparation Examples 145 to 288 can be prepared with an ordinary method by using 0.5 g of verosudil monohydrochloride in a free form instead of netarsudil dimesylate in the Preparation Examples 1 to 144.

[Preparation Examples 289 to 432] (Comparative)

Pharmaceutical preparations of Preparation Examples 289 to 432 can be prepared with an ordinary method by using 0.7 g of verosudil monohydrochloride in a free form instead of netarsudil dimesylate in the Preparation Examples 1 to 144.

[Preparation Examples 433 to 576] (Comparative)

Pharmaceutical preparations of Preparation Examples 433 to 576 can be prepared with an ordinary method by using 0.02 g of a compound represented by the formula (8) instead of netarsudil dimesylate in the Preparation Examples 1 to 144.

Industrial applicability

According to the present invention, a pharmaceutical preparation having excellent stability can be provided, and it can be suitably used in the pharmaceutical industry.

## Claims

1. Use of latanoprost for suppressing the content reduction of netarsudil or a salt thereof or a solvate thereof in a pharmaceutical preparation comprising an aqueous composition comprising the following components (A) and (B):

   (A) netarsudil or a salt thereof or a solvate thereof; and
   (B) latanoprost,

   wherein the aqueous composition is housed in a container formed of polypropylene.

2. Use of latanoprost for suppressing the content reduction of netarsudil or a salt thereof or a solvate thereof according to claim 1, wherein component (A) is netarsudil dimesylate.

## Patentansprüche

1. Verwendung von Latanoprost zur Unterdrückung der Gehaltsverringerung von Netarsudil oder einem Salz davon oder einem Solvat davon in einer pharmazeutischen Zubereitung, die eine wässrige Zusammensetzung umfasst, die die folgenden Komponenten (A) und (B) umfasst:

   (A) Netarsudil oder ein Salz davon oder ein Solvat davon und
   (B) Latanoprost,

   wobei die wässrige Zusammensetzung in einem Behälter aus Polypropylen untergebracht ist.

2. Verwendung von Latanoprost zur Unterdrückung der Gehaltsverringerung von Netarsudil oder einem Salz davon oder einem Solvat davon gemäß Anspruch 1, wobei es sich bei Komponente (A) um Netarsudildimesylat handelt.

## Revendications

1. Utilisation de latanoprost pour la suppression de la réduction de la teneur de nétadursil ou d’un sel correspondant ou d’un solvate correspondant dans une préparation pharmaceutique comprenant une composition aqueuse comprenant les composants suivants (A) et (B) :

(A) nétadursil ou un sel correspondant ou un solvate correspondant ; et
(B) latanoprost,

la composition aqueuse étant logée dans un récipient formé de polypropylène.

**2.** Utilisation de latanoprost pour la suppression de la réduction de la teneur de nétadursil ou d'un sel correspondant ou d'un solvate correspondant selon la revendication 1, le composant (A) étant le dimésylate de nétadursil.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2012525386 A
- WO 2009091898 A
- JP 2016515520 A
- EP 3590513 A1
- EP 3590514 A1
- EP 3590515 A1
- WO 2014144781 A1


### Non-patent literature cited in the description


- **BACHARACH J. et al.** *Ophthalmology,* 2015, vol. 122 (2), 302-7
- **STURDIVANT JM. et al.** *Bioorg Med Chem Lett.,* 2016, vol. 26 (10), 2475-80
- **WILLIAMS RD. et al.** *Am. J. Ophthalmol.,* 2011, vol. 152 (5), 834-41
- General Rules for Preparation. Japanese Pharmacopoeia